# EUROPÄISCHE PATENTANMELDUNG

(11) **EP 1 491 165 A1**
(43) Veröffentlichungstag der Anmeldung: **29.12.2004**
(21) Anmeldenummer: 04014709.2
(22) Anmeldetag: 23.06.2004
(51) Int. Cl.: A61F 2/44, A61F 2/30, A61F 2/46

(54) **Wirbelkörperprothese**

(30) Priorität: 23.06.2003 DE 10328307
(71) Anmelder: Rennebaum, Franz, 42109 Wuppertal (DE)
(72) Erfinder: Rennebaum, Franz, 42109 Wuppertal (DE)
(74) Vertreter: Füssel, Michael, Dipl.-Ing.

(57) **Zusammenfassung**

Es wird ein Implantat vorgeschlagen, das aus zueinander beabstandeten Widerlagerflanschen (5,6) besteht, die von einem Gewindestück (9) getragen werden, das an seinen Enden Gewindeabschnitte (12,13) in mit unterschiedlicher Gängigkeit oder Steigung aufweist. Das Gewindestück (9) ist drehfest mit einer Einstellscheibe (14) verbunden, durch deren Verdrehen eine Längenverstellung der Prothese (4) erfolgen kann. Zum Verdrehen der Einstellscheibe (14) sind umfänglich angeordnete Ausnehmungen vorgesehen, in die eine Drehmoment übertragende Klaue eingeführt werden kann. Gleichzeitig dienen die Ausnehmungen zur Aufnahme eines ich selbst auseinanderspreizenden Sicherungsstiftes (15), der mit Steckzapfen in Eingriffsbohrungen der Widerlagerflansche (5,6) eingreift. Zum Einsetzen der Prothese und zum Fixieren der Widerlagerflansche (5,6) beim Verdrehen der Einstellscheibe (14) dient eine zweiarmige Halterung mit abgewinkelten Eingriffszapfen, die verschwenkbar in die Eingriffsbohrungen der Widerlagerlflansche (5,6) einsetzbar ist. Auf diese Weise ist die Positionierung und Längeneinstellung der Wirbelkörperprothese (4) erleichtert.

## Beschreibung

Die Erfindung betrifft eine Wirbelkörperprothese für die menschliche Wirbelsäule, mit zwei zueinander beabstandeten, stirnseitigen Widerlagerflanschen zum Abstützen der Prothese gegen die benachbarten zwischenwirbelscheiben bzw. Wirbelkörper, wobei der Abstand zwischen den Stirnflächen der Widerlagerflansche einstellbar und in der eingestellten Position fixierbar ist.

Bei einer Beschädigung oder Zerstörung von Wirbelkörpern durch Unfall oder Knochenerkrankungen wie Osteoporose müssen die defekten Wirbelkörper durch künstliche Implantate ersetzt werden, damit die Stützfunktion der Wirbelsäule aufrecht erhalten werden kann.

Die einzusetzenden Prothesen müssen dabei paßgenau zwischen die benachbarten Wirbelkörper eingesetzt und die jeweils passende Länge eingestellt werden.

Das Einsetzen und Justieren der Wirbekörperprothesen erfordert dabei vom Operationspersonal eine erhebliche Geschicklichkeit, da derartige Operationen ventral, das heißt von der Bauchseite her durchgeführt werden müssen und die in der Bauchhöhle befindlichen Organe einen ungehinderten Zugriff auf die Operationsstelle erschweren. Eine dorsale, das heißt vom Rücken her durchzuführende Operation ist in der Regel nicht möglich, da in dieser Richtung das Rückenmark durch die Wirbellöcher der einzelnen Wirbel verläuft.

Gattungsgemäße Wirbelkörperprothesen sind beispielsweise aus der EP 0 904 751 A1, der EP 1 080 703 A2, der EP 0 975 288 B1 oder der WO 00/23013 bekannt. Die dem Stand der Technik entsprechenden Implantate lassen sich bezüglich ihrer Positionierung und Längeneinstellung bei der ventralen Operationsweise nur umständlich handhaben, so daß sich ein an die jeweilige Anatomie angepaßter Sitz unter Umständen nicht oder nur ungenügend erreichen läßt.

Der Erfindung liegt daher die Aufgabe zugrunde, eine Wirbelkörperprothese zu schaffen, deren Bauart eine vereinfachte Handhabung während der Operation ermöglicht, insbesondere die schnelle und feinfühlige Überbrückung aller praktisch vorkommenden Entfernungen zwischen den Wirbelkörpern ermöglicht.

Erfindungsgemäß wird diese Aufgabe gelöst durch die Merkmale des Hauptanspruchs.

Durch die erfindungsgemäße Bauart der Wirbelkörperprothese ist ein vergleichsweise Halten des Implantats zwischen den benachbarten Wirbelkörpern und währenddessen eine sehr genaue, an die jeweilige Anatomie angepaßte Längeneinstellung in kurzer Zeit möglich.

Der künstliche Wirbelkörper besteht im wesentlichen aus einem mittigen und für sich separaten Gewindestück, das an mindestens einem seiner beiden Enden einen Gewindeabschnitt trägt. Dabei steckt das Gewindestück mit dem Gewindeabschnitt im korrespondierenden Gewinde eines der beiden Widerlagerflansche. Bei Drehung des Gewindestücks wird der Widerlagerflansch - abhängig von der Drehrichtung - nach Außen oder nach Innen verschoben.

Das andere Ende des Gewindestücks steckt beispielsweise mit einem zylindrischen Zapfen in einer entsprechenden zylindrischen Bohrung des anderen Widerlagerflansches und ist dort drehbar gelagert.

Bei Drehung des Gewindestücks in der einen wie in der anderen Richtung ändert sich somit der Abstand zwischen den Außenflächen des Widerlagerflansches, die sich an dem benachbarten Wirbelkörpern abstützen.

Eine bevorzugte Ausführungsform sieht vor, das Gewindestück mit zwei Gewindeabschnitten zu versehen, von denen jeweils einer in einer zugehörigen Gewindebohrung des benachbarten Widerlagerflanschs steckt, so daß sich bei Drehung des Gewindestücks die Widerlagerflansche nach Außen bzw. Innen bewegen.

Hierzu ist vorgesehen, daß die beiderseitigen Gewindeabschnitte entweder unterschiedliche Gängigkeit oder unterschiedliche Gewindesteigung aufweisen. Auf die Gewindeabschnitte ist bei dieser Weiterbildung jeweils ein Widerlagerflansch aufgeschraubt, der ein an das jeweilige Gewinde des Gewindestückes angepaßtes Innengewinde aufweist. Auf diese Weise läßt sich - wie in allen anderen Ausführungsbeispielen - durch ein Verdrehen des auf dem Gewindestück angeordneten Trieborgans, welches erfindungswesentlich getrieblich beaufschlagbar ist, der Abstand zwischen den Widerlagerflanschen variabel verändern, sofern diese Flansche beim Verdrehen der Einstellscheibe fixiert werden. Das Einsetzen der Prothese und die Fixierung der Widerlagerflansche sowie das Verdrehen des Trieborgans erfolgt mit Hilfe zum Beispiel einer zweiarmigen Halterung bzw. einer zweiarmigen Klaue. Halterung und Klaue sind gleichzeitig aber lösbar an den Wirbelkörperprothese angebracht. Das Trieborgan kann zum Beispiel als Einstellscheibe ausgebildet sein.

Die Einstellscheibe weist zweckmäßigerweise eine Vielzahl von umfänglich angeordneten Ausnehmungen auf, die sich zum Eingreifen des erwähnten Drehmoment übertragenden Werkzeugs eignen. Gleichzeitig kann die Wirbelkörperprothese mit Hilfe dieser Ausnehmungen und eines Sicherungsstiftes bezüglich der eingestellten Länge sicher und dauerhaft fixiert werden. Dazu wird ein zusammendrückbarer Sicherungsstift in eine der Ausnehmungen der Einstellscheibe eingesetzt, der an seinen stirnseitigen Enden Steckzapfen aufweist, die in Eingriffsbohrungen auf der Innenseite der Widerlagerflansche eingreifen. In dieser Position ist ein Verdrehen der Einstellscheibe relativ zu den Widerlagerflanschen nicht mehr möglich, so daß sich die eingestellte Länge des Implantats nachträglich nicht mehr verändern kann.

Der verwendete Sicherungsstift besteht vorzugsweise aus einer Außenhülse und einer in der Außenhülse verschiebbar geführten Innenhülse. im Inneren der Hülsen befindet sich eine zusammendrückbare Feder, die den Sicherungsstift selbsttätig auseinander spreizt und dafür sorgt, daß er mit den Steckzapfen unverlierbar in den Eingriffsbohrungen der Widerlagerflanschen sitzt.

Zum Einsetzen des Sicherungsstiftes in die Ausnehmung der Einstellscheibe bzw. der Eingriffsbohrungen der Widerlagerflansche kann dieser manuell oder mit Hilfe eines geeigneten Werkzeuges zusammengedrückt werden.

Ein Auseinanderfallen des Sicherungsstiftes kann durch Rastringe oder dergleichen verhindert werden, die zwischen der Innen- und Außenhülse angeordnet sind.

Die Widerlagerflansche weisen an ihren Außenseiten vorzugsweise eine dreidimensionale Struktur auf, die beispielsweise aus dornenartigen Erhebungen besteht. Diese Struktur kann in die Außenseite der Widerlagerflansche eingearbeitet sein oder aus entsprechendne Profilscheiben bestehen, die an den Widerlagerflanschen befestigt sind.

Die durch die erfindungsgemäße Konstruktion der Wirbelkörperprothese ermöglichte einfache Handhabung ergibt sich zunächst beim Einsetzen des Implantats, zum Beispiel durch die gegenüberliegenden Eingriffsbohrungen in den Widerlagerflanschen, in die eine zusammendrückbare, sich selbsttätig aufspreizende zweiarmige Halterung mit abgewinkelten Eingriffszapfen eingesetzt werden kann. Auf diese Weise kann das Implantat ohne direktes Anfassen gehalten und positioniert werden. Gleichzeitig wird der obere Widerlagerflansch gegen ein unbeabsichtigtes Verdrehen relativ zum unteren Widerlagerflansch gesichert.

Zur exakten Längeneinstellung der Prothese wird nun die Einstellscheibe relativ zu den fixierten Widerlagerflanschen verdreht, wobei je nach Drehrichtung der Einstellscheibe ein Verlängern oder Verkürzen des künstlichen Wirbelkörpers möglich ist. Das Verdrehen der Einstellscheibe erfolgt dabei beispielsweise mit Hilfe der bereits erwähnten zweiarmigen Klaue, die mit ihren Armen in versetzt liegende Ausnehmungen der Einstellscheibe eingreift. Durch die Möglichkeit, die mit ihren Angriffszapfen in den Eingriffsbohrungen sitzende Halterung seitlich wegschwenken zu können, ist ein Verdrehen der Einstellscheibe mit Hilfe der Klaue problemlos möglich.

In einem weiteren Ausführungsbeispiel wird vorgesehen, daß die Einstellscheibe an ihrem Außenumfang eine Gewindeverzahnung mit axialer Komponente trägt.

Diese Axialverzahnung ist dazu bestimmt, mit einer verzahnten Schnecke zusammenzuwirken, die mittels eines geeigneten Halters vorübergehend formschlüssig an die Wirbelkörperprothese anlegbar und dann mittels eines Handgriffs drehbar ist.

Hierzu werden Ausführungsbeispiele angegeben.

Nachdem die Prothese auf diese Art und Weise exakt ausgerichtet und eingestellt wurde, wird die zweiarmige Halterung manuell zusammengedrückt und mit ihren abgewinkelten Eingriffszapfen aus den Eingriffsbohrungen entfernt. Anschließend wird die Klaue aus der Einstellscheibe herausgenommen und der Sicherungsstift in die miteinander fluchtenden Eingriffsbohrungen und die entsprechende umfängliche Ausnehmung der Einstellscheibe eingesetzt. Dies kann durch manuelles Zusammendrücken des Sicherungsstiftes, gegebenenfalls auch mit einem entsprechenden zangenartigen Werkzeug erfolgen.

Mit eingesetztem Sicherungsstift können sich die Widerlagerflansche und die Einstellscheibe nicht mehr relativ zueinander bewegen, so daß ein korrekter Sitz der Prothese in der Wirbelsäule gewährleistet ist.

Um die Eingriffsbohrungen in den Widerlagerflanschen mit den Ausnehmungen der Einstellscheibe fluchtend zueinander auszurichten, ist es zweckmäßig, wenn die Ausnehmungen und Angriffsbohrungen eine übereinstimmende umfängliche Winkellage aufweisen.

Die Abmessungen der Widerlagerflansche und des Gewindestückes sind vorzugsweise so gewählt, daß sich die Außenseiten der Widerlagerflansche bzw. Profilscheiben auf einen Abstand zwischen 30 mm und 45 mm einstellen lassen.

Die Erfindung ist in den Zeichnungen beispielsweise veranschaulicht und wird im Nachfolgenden anhand der Zeichnung im Einzelnen erläutert. Es zeigen:
- Fig.1: eine schematische Schnittdarstellung durch den Lendenwirbelbereich einer Wirbelsäule mit einer eingesetzten Wirbelkörperprothese
- Fig.2: einen Schnitt durch eine längeneinstellbare Wirbelkörperprothese mit unterschiedlichen Gewindegängigkeiten des Gewindestückes,
- Fig.3: einen Schnitt durch eine Wirbelkörperprothese gemäß Fig.2 in verlängertem und gesichertem Zustand,
- Fig.4: eine Draufsicht auf die Einstellscheibe der Wirbelkörperprothese gemäß Fig.2 und 3,
- Fig.5: einen Schnitt durch die Wirbelkörperprothese gemäß Fig.2 mit einer eingesetzten Halterung,
- Fig.6: einen Schnitt durch die Wirbelkörperprothese gemäß Fig.2 mit einer eingesetzten Klaue zum Verstellen der Einstellscheibe mit unterschiedlichen Gewindesteigungen,
- Fig.7: eine Draufsicht auf die Einstellscheibe gemäß Fig.6 mit eingesetzter Klaue,
- Fig.8: ein Ausführungsbeispiel mit Madenschrauben als Umfangssicherung,
- Fig.9: Aufsicht auf die Einstellscheibe eines weiteren Ausführungsbeispiels,
- Fig.10: Ausführungsbeispiel in Ansicht von oben mit Verstellmöglichkeit durch Axialverzahnung -Schnecke,
- Fig.11: vereinfachtes Ausführungsbeispiel gemäß Fig.10,
- Fig.12: Ausführungsbeispiel gemäß Fig.11 in seitlicher Ansicht,
- Fig.13: Ausführungsbeispiel gemäß Fig.12 während des Einstellvorgangs,
- Fig.14: ein weiteres Ausführungsbeispiel,
- Fig.15: eine weitere Ansicht des Ausführungsbeispiels gemäß Fig.14, und
- Fig.16: noch eine Ansicht des Ausführungsbeispiels gemäß Fig.14.

Sofern im folgenden nichts anderes gesagt ist, gilt die folgende Beschreibung stets für alle Figuren.

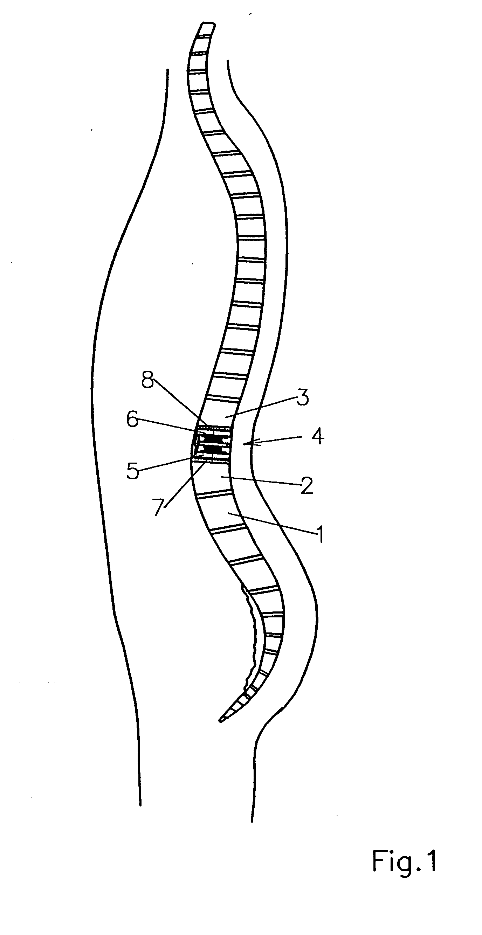

Bei der Fig.1 dargestellten Seitenansicht sind die Wirbelkörper 1 des ersten, 2 des zweiten und 3 des vierten Lendenwirbels dargestellt, wobei aus Gründen der Übersichtlichkeit auf die Wiedergabe der das Wirbelloch umgebenden Wirbelbögen und der Dornfortsätze, die sich in der Zeichnung gesehen nach rechts erstrecken, verzichtet wurde.

Zwischen dem Wirbelkörper 2 und 3 befindet sich eine Wirbelkörperprothese 4, die sich mit Hilfe von Widerlagerflanschen 5,6 gegen die Zwischenwirbelscheibe 7 des zweiten Lendenwirbels bzw. die Zwischenwirbelscheibe 8 des vierten Lendenwirbels abstützt.

Die Widerlagerflansche 5,6 werden von einem Gewindestück 9 getragen, das - hier - an seinem oberen Ende 10 und seinem unteren Ende 11 Gewindeabschnitte 12,13 mit unterschiedlicher Gängigkeit trägt.

Anstelle eines Gewindestücks, welches Gewindeabschnitte 12,13 unterschiedlicher Gängigkeit besitzt, kann auch ein Gewindestück mit Gewindeabschnitten unterschiedlicher Gewindesteigungen bei übereinstimmender Gängigkeit verwendet werden.

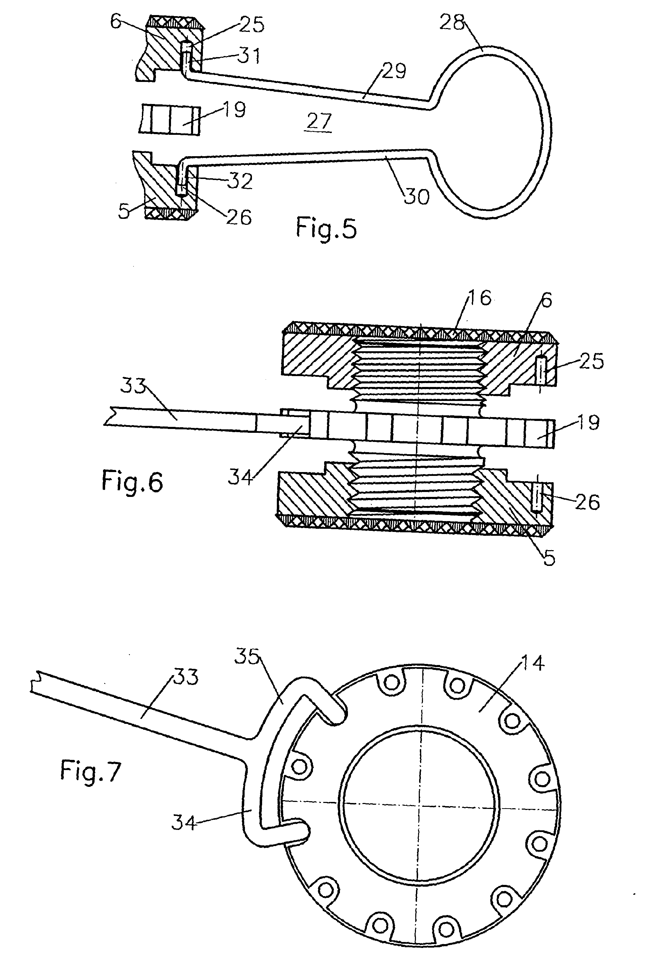

Dieser Fall ist in Fig. 6 dargestellt.

Dort ist die Steigung des oberen Gewindeabschnitts 12 geringer als die Steigung des unteren Gewindeabschnitts, so daß sich bei Drehung in der einen sowie in der anderen Richtung die Endflächen der Widerlagerflansche entsprechend auseinander oder aufeinander zu bewegen.

Der Vollständigkeit halber soll erwähnt sein, daß lediglich ein einzelner Gewindeabschnitt notwendig ist, während das andere Ende des Gewindeabschnitts mit einem zylindrischen Bolzen in einer zylindrischen Bohrung des zugehörigen Widerlagerflanschs drehbar gelagert ist, so daß sich bei entsprechender Drehung des Gewindestücks ebenfalls die Abstützflächen der Widerlagerflansche voneinander weg bzw. aufeinander zu bewegen.

Will man bei kleinstmöglichem Drehwinkel die größtmögliche Abstandsänderung erzielen, so empfiehlt es sich, eines der Gewinde als Rechtsgewinde und das andere Gewinde als Linksgewinde auszubilden, so daß durch diese unterschiedliche Gängigkeit nur geringe Drehwinkel notwendig sind, um die gewünschte Abstandsänderung herbeizuführen.

In der Mitte des Gewindestückes 9 befindet sich eine Einstellscheibe 14, mit deren Hilfe der Abstand zwischen den Widerlagerflanschen 5,6 eingestellt werden kann.

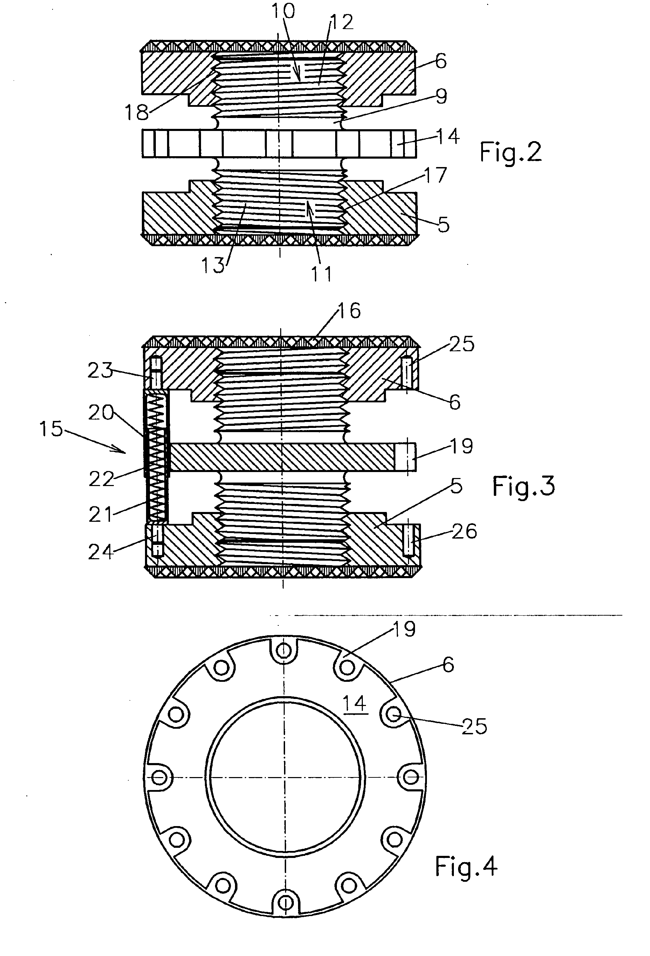

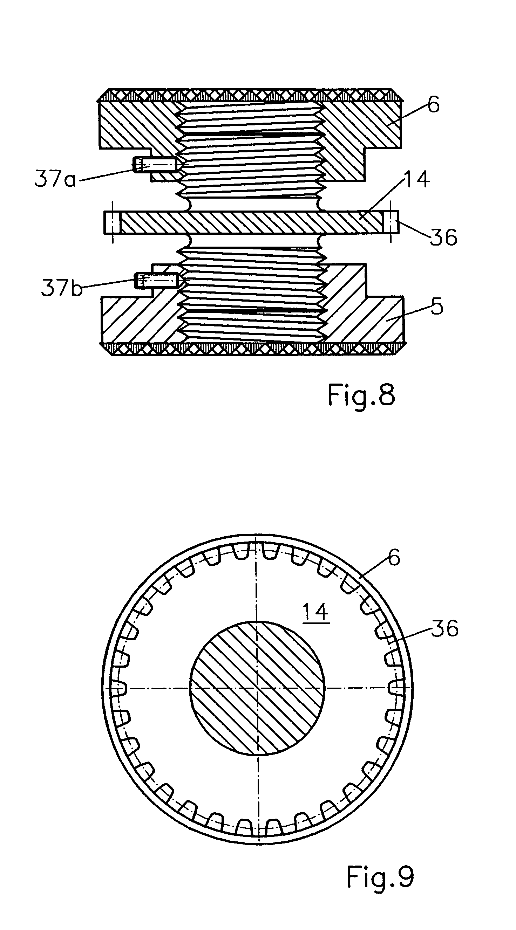

Eine Arretierung der Einstellscheibe 14 und damit eine Fixierung der Widerlagerflansche 5,6 erfolgt z.B. mit Hilfe eines Sicherungsstiftes 15 (Fig.3) oder mit Hilfe zweier radialer Klemmschrauben 37a,b (Fig.8), die mit jeweils einem der Widerlagerflansche zusammenwirken.

Die Widerlagerflansche 5,6 weisen an ihren Außenseiten eine dreidimensionale Struktur mit dornenartigen Erhebungen 16 auf.

Bei der Schnittdarstellung gemäß Fig.2 ist die Wirbelkörperprothese 4 in nicht arretiertem Zustand vergrößert dargestellt. Insbesondere ist hier zu erkennen, daß das Gewindestück 9 an seinen Enden Gewindeabschnitte 12,13 trägt, die eine unterschiedliche Gängigkeit aufweisen. Der obere Widerlagerflansch 5 weist ein Innengewinde 17 auf, das den Gewindeabschnitt 12 aufnimmt, während der Widerlagerflansch 6 mit seinem Innengewinde 18 den Gewindeabschnitt 13 aufnimmt.

Die fest mit dem Gewindestück 9 verbundene Einstellscheibe 14 weist eine Vielzahl von regelmäßig über ihren äußeren Umfang verteilten Ausnehmungen 19 auf, die sowohl zum Verdrehen der Einstellscheibe 14 relativ zu den Widerlagerflanschen 5,6 wie auch zum Einsetzen des Sicherungsstiftes 15 dienen.

Dieser im Einzelnen in Fig.3 gezeigte Sicherungsstift 15 besteht im wesentlichen aus einer Außenhülse 20, in der eine Innenhülse 21 verschiebbar geführt ist. In den Hülsen 20,21 ist eine Schraubenfeder 22 angeordnet, die den Sicherungsstift 15 selbsttätig auseinander spreizt und mit Hilfe von Steckzapfen 23,24, die in Eingriffsbohrungen 25,26 der Widerlagerflansche 5,6 eingreifen, in der dargestellten Position fixiert.

Der Sicherungsstift 15 sitzt dabei in einer der bereits erwähnten Ausnehmungen 19 der Einstellscheibe 14, so daß die Einstellscheibe 14 relativ zu den Widerlagerflanschen 5,6 arretiert ist, wobei die Ausnehmung 19 und die Eingriffsbohrungen 25,26 miteinander fluchten. Die entsprechende Position ist im Einzelnen in der Draufsicht gemäß Fig.4 dargestellt, wo die Einstellscheibe 14, die Eingriffbohrungen 26 und die Ausnehmungen 19 zu erkennen sind.

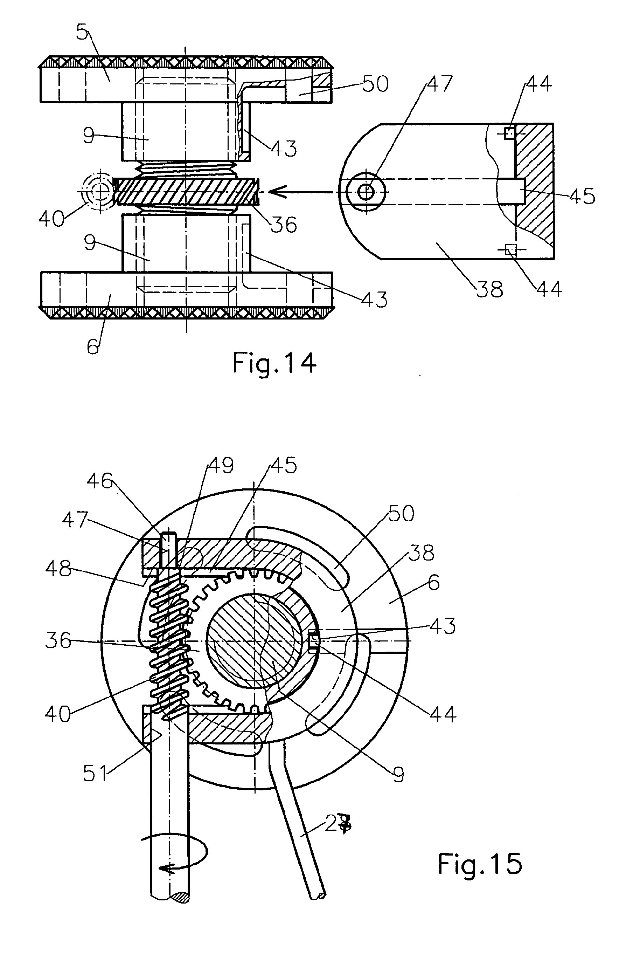

Um die Wirbelkörperprothese 4 leichter an Ort und Stelle von der Bauchseite her in die Wirbelsäule einsetzen zu können, wird zum Beispiel die in Fig.5 oder 15 schematisch dargestellte, zweiarmige Halterung 27 verwendet. Die Halterung 27 gemäß Fig.5 weist einen elastisch verformbaren Griff 28 und die Arme 29,30 auf, deren Enden umgebogen sind und die Eingriffszapfen 31,32 bilden, die in die Eingriffsbohrungen 25,26 der Widerlagerflansche 5 beziehungsweise 6 eingeführt werden können. Auf diese Weise kann die Wirbelkörperprothese 4 an Ort und Stelle positioniert werden, ohne unmittelbar vom Operateur berührt zu werden. Die Halterung 38 gemäß Fig.15 besteht im wesentlichen aus einem U-förmigen Gehäuseteil, an welchem der Griff sitzt, vorzugsweise an einer fest angebrachten Haltestange 27. Die parallelen Schenkel des U sind über die Mitte des Gewindestücks 9 hinaus verlängert und weisen miteinander fluchtende Bohrungen 47,51 auf.

Das Durchsteckloch 51 dient dazu, die Verstellschnecke 40 so weit in Richtung zu der Verzahnung 36, die sich am Umfang der Einstellscheibe 14 befindet, zu verlagern, daß dort der erfindungsgemäße getriebliche Eingriff zum Antrieb des Trieborgans 14 entsteht.

Zu diesem Zweck ist in diesem Schenkel des U eine Durchgangsbohrung 51 angebracht, deren Durchmesser größer als der Außendurchmesser der Verstellschnecke 40 ist.

Das bei der Positionierung der Verstellschnecke 40 vorausgehende Ende weist hingegen einen Führungszapfen 46 auf, dessen Durchmesser an den Durchmesser der Führungsbohrung 47 angepaßt ist, welche sich im anderen Schenkel des U befindet.

An diesem Ende besitzt die Verstellschnecke 40 eine Durchmesserstufe 49, welche auf eine an der Innenseite des U ausgebildete Gegenfläche stößt, die als Tiefenanschlag 48 wirkt.

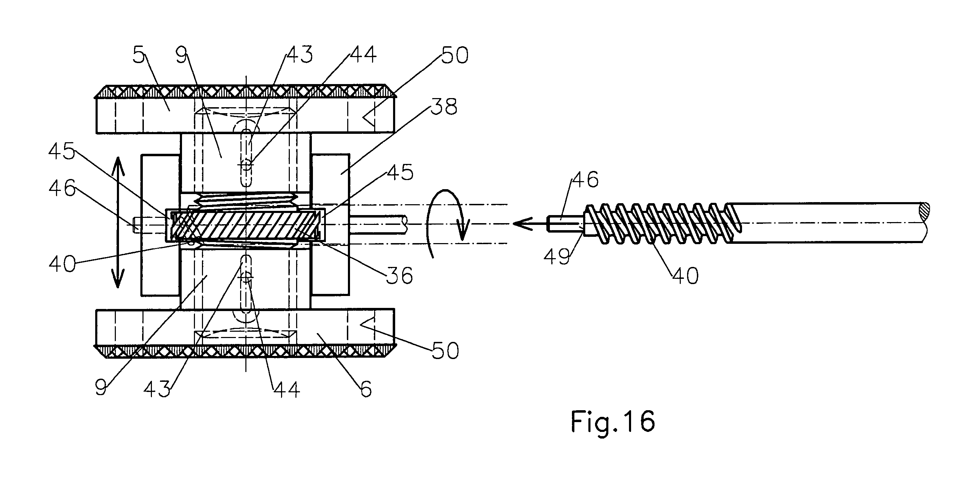

Wie insbesondere aus den Fig.14 bis 16 hervorgeht, ist der Außendurchmesser der Einstellscheibe 14 deutlich geringer als der Außendurchmesser der Widerlagerflansche 5,6.

Auf diese Weise läßt sich die U-förmige Halterung 38 ohne Verlagerung des umgebenden Gewebes in die Fixierposition zwischen den Wirbelkörpern verbringen, wobei dies vorzugsweise mit eingesteckter Verstellschnecke 40 vorgenommen wird.

Um eine möglichst kompakte Baugruppe zu erzielen, weist die U-förmige Halterung 38 eine Umfangsnut 45 auf, die mit geringem Spiel zur Verzahnung 36 positioniert ist, sobald die Wirbelkörperprothese 4 eingeführt werden soll.

Zusätzlich sind am Innenumfang des Verbindungsbogens zwischen den beiden Schenkeln des U beidseits der Umfangsnut 45 Fixierstifte 44 vorgesehen, die in der Montageposition gemäß Fig.14 bis 16 in Richtung zur zentralen Mitte der Wirbelkörperprothese zeigen.

Dort sind an den Widerlagerflanschen 5,6 Fixiernuten 43,44 vorhanden, in welche die Fixierstifte 44 eingreifen, so daß eine umfangsmäßige Fixierung der Wirbelkörperprothese in Einbauposition gegeben ist.

Die Längserstreckung der Fixiernuten 43 ist dabei in Richtung zur Einstellscheibe 14 begrenzt, so daß hiermit auch die maximal mögliche Axialposition der Wirbelkörperprothese beschränkt ist.

Gemäß Fig.5 dient ebenfalls gleichzeitig die Halterung 27 dazu, die Eingriffsbohrungen 25,26 fluchtend zueinander auszurichten und die Widerlagerflansche 5,6 festzuhalten, so daß sich die Einstellscheibe 14 relativ dazu verdrehen und damit die gewünschte Länge der Wirbelkörperprothese 4 einstellen läßt.

Das Verdrehen der Einstellscheibe 14 erfolgt z.B. mit Hilfe einer zweiarmigen Klaue 33, die mit ihren Armen 34,35 in versetzt liegende Ausnehmungen 19 der Einstellscheibe 14 eingreift. (Vergleiche Fig.6 und 7)

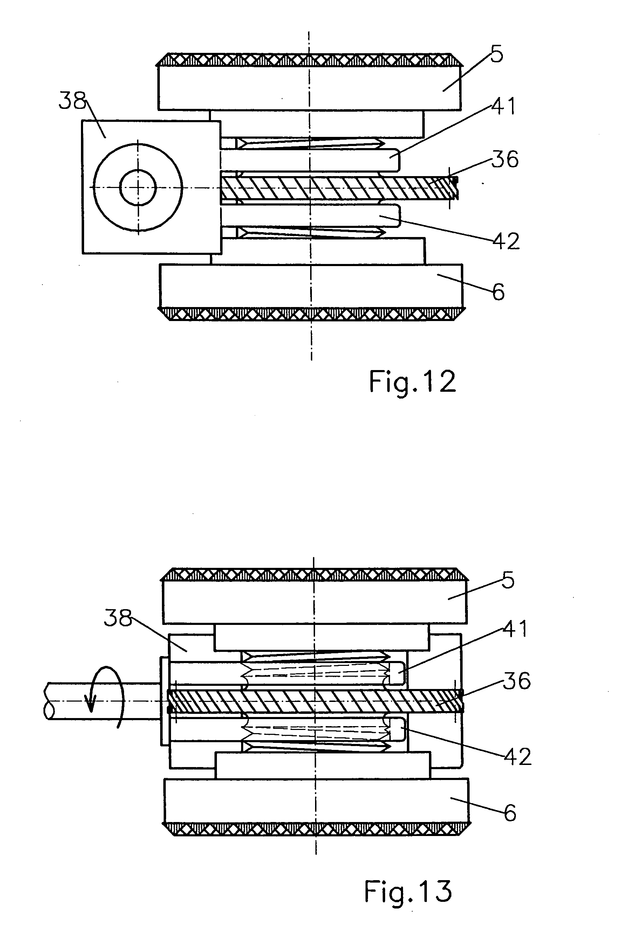

Darüber hinaus zeigen die Fig.8 bis 13 eine Weiterbildung, bei welcher das Verdrehen des Gewindestücks 9 über das Zusammenwirken der Axialverzahnung, die am Außenrand der Einstellscheibe 14 angebracht ist, und einer passenden Gewindeschnecke 40 erfolgt. Die Gewindeschnecke 40 wird drehbar in einer zugeordneten Halterung 38 gelagert. Die Halterung 38 wird zum Zwecke der Einstellung des Gewindestücks mittels einer zweiarmigen Klaue an das Gewindestück angelegt und ist somit bezüglich der Wirbelkörperprothese 4 fixiert. In eine entsprechende Bohrung 39 wird dann die Verstellschnecke 40 eingeführt, die mit der am Außenumfang der Einstellscheibe befindlichen Axialverzahnung 36 kämmt.

Bei Drehung der Verstellschnecke 40 in der entsprechenden Richtung bewegen sich die Widerlagerflansche 5,6 auseinander oder aufeinander zu.

Danach kann die Halterung 38 aus ihrer Anlageposition an der Wirbelkörperprothese 4 entfernt werden und dieser Teil der Operation ist beendet.

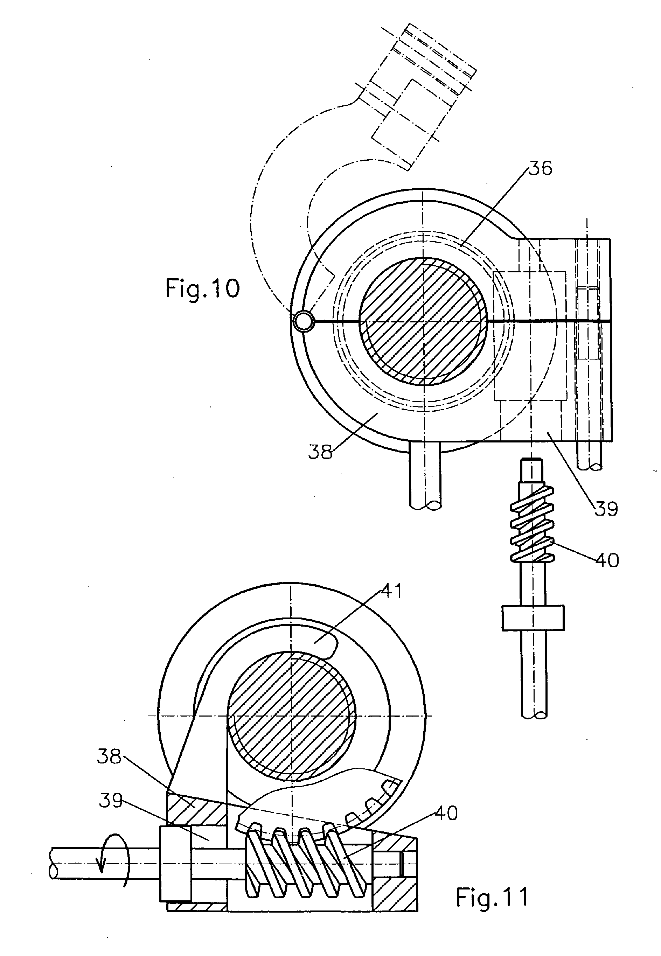

Im Vergleich hierzu zeigt Fig.10 die Möglichkeit, die Halterung 38 zweiteilig auszubilden und über ein Scharnier miteinander zu verbinden.

Bei diesem Ausführungsbeispiel läßt sich die Halterung vollständig um die Wirbelkörperprothese 4 herumlegen, so daß während des Drehvorgangs der Verstellschnecke 40 die Halterung 38 nicht gesondert festgehalten werden muß.

Hierzu zeigen insbesondere die Fig.3,12 und 13 ein Ausführungsbeispiel, bei welchem die Halterung 38 über zwei Klauen 41,42 verfügt, die zwischen sich einen Abstand bilden, der so groß ist, daß die Einstellscheibe 14 zwanglos zwischen die Klauen paßt, gegebenenfalls hier sogar eine Führung bietet.

Ein Verdrehen der Einstellscheibe 14 mit Hilfe der Klaue 33 oder der Verstellschnecke 40 trotz eingesetzter Halterung 27 ist problemlos möglich, da die Halterung seitlich weggeschwenkt werden kann.

Nachdem auf die beschriebene Weise die Positionierung der Wirbelkörperprothese 4 zwischen den benachbarten Wirbelkörpern 2 und 3 erfolgt und die richtige Länge eingestellt wurde, wird, wie zuvor beschrieben, der Sicherungsstift 15 eingesetzt, so daß die Wirbelkörperprothese 4 unbeabsichtigt nicht mehr verstellt werden kann.

Anstelle des Sicherungsstifts 15 können auch gemäß Ausführungsbeispiel Fig.8 die beiden Klemmschrauben 37a,37b angezogen werden. Diese Klemmschrauben 37a,37b sitzen in entsprechenden Gewindebohrungen, die Bestandteil der Widerlagerflansche 5,6 sind und weisen an ihren nach Innen gerichteten Enden eine Spitze auf, die sich beim entsprechenden Anzugsmoment in die Gewindezapfen des Gewindestücks eingräbt und auf diese Weise die beiden Widerlagerflansche 5,6 in Umfangsrichtung am Gewindestück 9 fixiert.

Die Widerlagerflansche können mit Durchbrüchen 50 versehen sein, um ein Hineinwachsen des angrenzenden Gewebes in die Wirbelkörperprothese zu ermöglichen.

### Bezugszeichenliste:

- 1: Wirbelkörper
- 2: Wirbelkörper
- 3: Wirbelkörper
- 4: Wirbelkörperprothese
- 5: Widerlagerflansch
- 6: Widerlagerflansch
- 7: Zwischenwirbelscheibe
- 8: Zwischenwirbelscheibe
- 9: Gewindestück
- 10: oberes Ende
- 11: unteres Ende
- 12: Gewindeabschnitt
- 13: Gewindeabschnitt
- 14: Einstellscheibe
- 15: Sicherungsstift
- 16: Erhebung
- 17: Innengewinde
- 18: Innengewinde
- 19: Ausnehmung
- 20: Außenhülse
- 21: Innenhülse
- 22: Schraubenfeder
- 23: Steckzapfen
- 24: Steckzapfen
- 25: Eingriffsbohrung
- 26: Eingriffsbohrung
- 27: Halterung
- 28: Griff
- 29: Arm
- 30: Arm
- 31: Eingriffszapfen
- 32: Eingriffszapfen
- 33: Klaue
- 34: Arm
- 35: Arm
- 36: Verzahnung
- 37a: Klemmschraube
- 37b: Klemmschraube
- 38: Halterung
- 39: Bohrung
- 40: Verstellschnecke
- 41: Klaue
- 42: Klaue
- 43: Fixiernut
- 44: Fixierstift
- 45: Umfangsnut
- 46: Führungszapfen
- 47: Führungsbohrung
- 48: Tiefenanschlag
- 49: Durchmesserstufe
- 50: Durchbruch
- 51: Durchsteckloch

## Patentansprüche

1. Wirbelkörperprothese für die menschliche Wirbelsäule, mit zwei zueinander beabstandeten, stirnseitigen Widerlagerflanschen (5,6) zum Abstützen der Prothese gegen die benachbarten Zwischenwirbelscheiben bzw. Wirbelkörper, wobei der Abstand zwischen den Stirnflächen der Widerlagerflansche (5,6) einstellbar und in der eingestellten Position fixierbar ist, **dadurch gekennzeichnet, daß** zumindest einer der Widerlagerflansche (5,6) mit einer Gewindebohrung (12,13) auf dem Ende (10,11) eines von beiden Widerlagerflanschen separaten Gewindestückes (9) sitzt welches sich zwischen den Widerlagerflanschen (5,6) befindet und welches an einem zwischen den Widerlagerflanschen befindlichen und von außen zugänglichen Umfangsbereich ein getrieblich beaufschlagbares Trieborgan (14) aufweist und das mittels des Trieborgans (14) relativ zu beiden Widerlagerflanschen (5,6) verdrehbar ist während gleichzeitig eine Halterung (38) an der Wirbelkörperprothese lösbar befestigt ist, und daß vorzugsweise das Gewindestück relativ zu beiden Widerlagerflanschen in Drehrichtung fixierbar ist.

2. Wirbelkörperprothese nach Anspruch 1, **dadurch gekennzeichnet, daß** beide Widerlagerflansche (5,6) mit jeweils einer Gewindebohrung (12,13) und daß das Gewindestück (9) mit korrespondierenden Gewindezapfen versehen ist.

3. Wirbelkörperprothese nach Anspruch 2, **dadurch gekennzeichnet, daß** die Gewindezapfen unterschiedliche Gängigkeit aufweisen.

4. Wirbelkörperprothese nach Anspruch 2, **dadurch gekennzeichnet, daß** die Gewindezapfen unterschiedliche Gewindesteigung aufweisen.

5. Wirbelkörperprothese nach Anspruch 1, **dadurch gekennzeichnet, daß** das Gewindestück (9) lediglich einen Gewindezapfen aufweist und an dem gegenüberliegenden Ende mit einem zylindrischen Zapfen in einer zylindrischen Bohrung des zugeordneten Widerlagerflansches steht.

6. Wirbelkörperprothese nach einem der Ansprüche 1 bis 5, **dadurch gekennzeichnet, daß** das Trieborgan als zwischen den endseitigen Gewindeabschnitten (12,13) des Gewindestückes (9) angeordnete, drehfest mit dem Gewindestück (9) verbundene, sich parallel zu den Widerlagerflanschen (5,6) erstreckende Einstellscheibe (14) ausgebildet ist.

7. Wirbelkörperprothese nach Anspruch 6, **dadurch gekennzeichnet, daß** die Einstellscheibe (14) eine Vielzahl von umfänglich angeordneten Ausnehmungen (19) zum Eingreifen eines Drehmoment übertragenden Werkzeuges aufweist.

8. Wirbelkörperprothese nach Anspruch 7, **dadurch gekennzeichnet, daß** die umfänglich angeordneten Ausnehmungen (19) als Rasteinrichtung zum Arretieren eines gegen die Kraft einer Feder zusammendrückbaren, sich selbständig auseinanderspreizenden Sicherungsstiftes (15) gegen ein Verdrehen der Widerlagerflansche (5,6) ausgebildet sind und daß die Widerlagerflansche (5,6) auf ihren einander zugewandten Innenseiten Eingriffsbohrungen (25,26) zur Aufnahme von endseitigen Steckzapfen (23,24) des Sicherungsstiftes (15) aufweisen.

9. Wirbelkörperprothese nach Anspruch 8, **dadurch gekennzeichnet, daß** der Sicherungsstift (15) eine Außenhülse (20) und eine in der Außenhülse (20) verschiebbar geführte Innenhülse (21) aufweist, daß die Steckzapfen (23,24) zum Einsetzen in die Eingriffsbohrungen (25,26) der Widerlagerflansche (5,6) stirnseitig an der Außenhülse (20) bzw. Innenhülse (21) angeordnet sind und daß sich die zusammendrückbare Feder (22) zum selbsttätigen Aufspreizen des Sicherungsstiftes (15) mit ihren Enden gegen die inneren Stirnflächen der Außenhülse (20) und der Innenhülse (21) abstützt.

10. Wirbelkörperprothese nach Anspruch 9, **dadurch gekennzeichnet, daß** die Innenhülse (21) des Sicherungsstiftes (15) gegen ein Herausfallen aus der Außenhülse (20) gesichert ist.

11. Wirbelkörperprothese nach Anspruch 1 bis 8, **dadurch gekennzeichnet, daß** die Widerlagerflansche (5,6) stirnseitig angeordnete Profilscheiben tragen.

12. Wirbelkörperprothese nach Anspruch 11, **dadurch gekennzeichnet, daß** die Profilscheiben auf ihren Außenseiten dornenartige Erhebungen (16) aufweisen.

13. Wirbelkörperprothese nach einem der Ansprüche 1 bis 12, **dadurch gekennzeichnet, daß** zum ventralen Einsetzen der Prothese (4) in eine menschliche Wirbelsäule eine zusammendrückbare, sich selbsttätig aufspreizende zweiarmige Halterung (27) mit abgewinkelten Eingriffszapfen (31,32) zum verschwenkbaren Eingreifen in Eingriffsbohrungen (25,26) der Widerlagerflansche (5,6) vorgesehen ist, die sich in etwa fluchtend gegenüberliegen.

14. Wirbelkörperprothese nach einem der Ansprüche 6 bis 13, **dadurch gekennzeichnet, daß** zum Verdrehen des Gewindestückes (9) relativ zu den Widerlagerflanschen (5,6) eine an einem Haltegriff befestigte, zweiarmige Klaue (33) vorgesehen ist, die mit ihren Armen (34,35) jeweils in versetzt liegende Ausnehmungen (19) der Einstellscheibe (14) eingreift.

15. Wirbelkörperprothese nach einem der Ansprüche 1 bis 14, **dadurch gekennzeichnet, daß** zum Verdrehen des Gewindestücks (9) die Einstellscheibe (14) an ihrem Außenumfang eine Verzahnung aufweist, die zum Zusammenwirken mit einer verzahnten Schnecke bestimmt ist, welche mittels eines Halters vorübergehend formschlüssig an die Wirbelkörperprothese anlegbar und dann mittels eines Handgriffs drehbar ist.

16. Wirbelkörperprothese nach einem der Ansprüche 1 bis 15, **dadurch gekennzeichnet, daß** die Ausnehmungen (19) der Einstellscheibe (14) und die Eingriffsbohrungen (25,26) in den Widerlagerflanschen (5,6) eine übereinstimmende umfängliche Winkellage aufweisen.

17. Wirbelkörperprothese nach einem der Ansprüche 1 bis 16, **dadurch gekennzeichnet, daß** der Abstand zwischen den Außenseiten der Widerlagerflansche (5,6) bzw. Profilscheiben zwischen 30 mm und 45 mm einstellbar ist.
